# EUROPÄISCHE PATENTSCHRIFT

(11) **EP 2 356 600 B1**
(45) Veröffentlichungstag und Bekanntmachung des Hinweises auf die Patenterteilung: **20.09.2017**
(21) Anmeldenummer: 09748996.7
(22) Anmeldetag: 21.10.2009
(51) Int. Cl.: G06F 19/00, H04L 29/06, G06F 13/10, G06F 3/048

(54) **PATIENTENVERWALTUNGSSYSTEM MIT INTELLIGENTER SCHNITTSTELLENEINRICHTUNG ZUR ÜBERTRAGUNG MEDIZINISCHER DATEN**
PATIENT ADMINISTRATION SYSTEM COMPRISING INTELLIGENT INTERFACE DEVICE FOR THE TRANSMISSION OF MEDICAL DATA
SYSTÈME DE GESTION DE PATIENTS COMPORTANT UN DISPOSITIF INTERFACE INTELLIGENT POUR LA TRANSMISSION DE DONNÉES MÉDICALES

(30) Priorität: 18.11.2008 DE 102008057910
(43) Veröffentlichungstag der Anmeldung: 17.08.2011
(73) Patentinhaber: MicroNova AG, 85256 Vierkirchen (DE)
(72) Erfinder: LINGMANN, Jürgen, 85221 Dachau (DE)
(74) Vertreter: Grünecker Patent- und Rechtsanwälte PartG mbB
(86) Internationale Anmeldenummer: PCT/EP2009/007538
(87) Internationale Veröffentlichungsnummer: WO 2010/057557

(56) Entgegenhaltungen:
- EP-A1- 1 850 342
- EP-A2- 1 416 419
- EP-A2- 1 471 454
- WO-A1-01/46016
- WO-A2-02/084531
- WO-A2-2005/057465
- WO-A2-2006/044518
- DE-A1- 10 050 087
- DE-A1-102006 042 317
- US-A- 5 099 444
- US-A- 5 261 079
- US-A- 6 049 794
- US-A1- 2003 060 688
- US-A1- 2008 263 050
- US-B1- 6 456 277

## Beschreibung

Die vorliegende Erfindung bezieht sich auf ein Patientenverwaltungssystem und -verfahren, sowie einer zugehörigen Schnittstelleneinrichtung zur verbesserten Kommunikation mit einer Patientenverwaltungssoftware.

### Hintergrund der Erfindung

In Krankenhäusern, Kliniken oder Arztpraxen kommen sogenannte Patientenverwaltungssysteme zum Einsatz, die auf einem Computer implementiert sind. Diese Patientenverwaltungssysteme dienen zur Erfassung personenbezogener Grund-Daten, wie Name, Vorname(n), Geburtsdatum, Geschlecht, Krankenkasse sowie einer eindeutigen Patientennummer. Des Weiteren können Anamnesen, Befunde, Diagnosen, Therapien und weitere Prozedere erfasst werden. Darüber hinaus ist es möglich, aus diesen Patientenverwaltungssystemen beispielsweise Rezepte und Überweisungen zu erzeugen und auszudrucken.

Zum Datenaustausch zwischen einem medizinischen Gerät, wie beispielsweise ein Röntgen- oder Ultraschallgerät, gibt es hierfür entworfene Datenschnittstellen. Anfänglich dienten physische Datenträger wie beispielsweise Disketten dazu, die Daten von einem Gerät zu einem anderen zu transportieren, diese wurden aber dann durch andere Kommunikationsmedien, wie beispielsweise kabelgebundene oder kabellose Kommunikation über Netzwerkverbindungen, verdrängt. Diese Datenschnittstellen basieren auf einem definierten Standard, wie beispielsweise dem ADT-, GDT-, BDT- oder HL7-Standard. Diese Schnittstellen sind bei der jeweiligen Endbenutzersoftware, d.h. bei dem jeweiligen Patientenverwaltungssystem-Programm, unterschiedlich implementiert, da nur wenige der Schnittstellenparameter verbindlich definiert sind (sogenannte Muss-Felder). So ist bei der GDT-Schnittstelle lediglich die Übergabe des Patientennamens, Vornamens, Geburtsdatum, Geschlecht, Krankenkasse und Patientennummer zwingend erforderlich. Darüber hinaus sind in der Regel noch sehr viele weitere optionale Felder definiert oder definierbar. Ein Beispiel für ein solches optionales Feld ist ein Feld für die Übergabe von Blutdruckwerten.

Da sich der jeweilige Programmierer bei dem Entwerfen und Programmieren eines Patientenverwaltungsprogramms in der Regel nur an die zwingend erforderlichen Muss-Felder hält und optionale Felder (sogenannte Kann-Felder) nach Belieben oder Notwendigkeit definiert und auch wieder um-definiert, ist der Datenaustausch zwischen Patientenverwaltungssystemen von unterschiedlichen Anbietem schwierig bis unmöglich.

Ein solches Patientenverwaltungssystem ist aus der DE 100 50 087 A1 bekannt. Auf einem Zentralrechner wird hier eine Datenbank bereitgestellt, auf dem bereits erfasste Daten gespeichert werden, darunter auch Daten, die durch medizinische Messgerate gewonnen wurden. An diesen Zentralrechner angeschlossene Ärzte können diese Daten dann auf ihrem PC abrufen. Das Ziel der in diesem Dokument beschriebenen Technik liegt in der Verbesserung der Organisation und Kommunikation zwischen verstreut liegenden Arztpraxen.

Um die zuvor beschriebene Hürde beim Datenaustausch zu überwinden, gibt es bereits zahlreiche Kooperationen, die sich das Ziel gesetzt haben, eine einheitliche Definition der Schnittstelle zum direkten Datenaustausch zu erarbeiten. Aber die einheitliche Definition solch einer Schnittstelle ist mit der Schwierigkeit verbunden, dass, sobald sich die Struktur einer der Schnittstellen im Rahmen einer Aktualisierung ändert oder gar neu gestaltet wird, jegliche Änderungen übereinstimmend in allen Schnittstellen implementiert und auf Kompatibilität überprüft werden müssen. Dies hat den Nachteil, dass ein Großteil des Aufwandes nicht auf die Weiterentwicklung der Schnittstelle abzielen kann, sondern auf eine Prüfung und Sicherstellung der Schnittstellen untereinander verwendet werden muss.

US 5,099,444 bezieht sich auf Datengewinnung, ohne dass diese Daten direkt eingegeben werden müssen. Ein Mikroprozessor wandelt einen ASCII-Text in Tastatur-Codes um.

US 6,456,277 B1 bezieht sich auf eine Datenumwandlung zwischen unterschiedlichen Tastaturtypen. Eine erste Scan-Code-Gruppe für eine erste Tastatur wird in eine zweite Scan-Code-Gruppe für eine zweite Tastatur umgewandelt.

US 5,261,079 beschreibt ein Computer-System welches eine Tastatur umfasst, die einen Tastatur-Gerätetreiber umfasst. Weitere Arten von Eingabegeräten, wie Handabdruck-Terminals, Licht Kugelschreiber, etc., können an Stelle der Tastatur eingesetzt werden. Eine Tastatur-Gerätetreiber-Emulator wandelt Eingabedaten aus dem alternativen Eingabegerät um so dass die Daten als Tastatur erzeugte Daten erscheinen.

US 2008/0263050 A1 beschreibt ein computer-implementiertes Verfahren zur Verwaltung von Daten für eine klinische Entscheidungssystem und umfasst das Bereitstellen eines Computer-Management-System bestehend aus einem Server und einer Datenbank und das Herunterladen des Entscheidungssystems auf das Computer-Management-System. Das Entscheidungssystem umfasst eine Vielzahl von Regeln und empfängt Daten.

US 6,049,794 beschreibt ein System für das Screening einer medizinischen Entscheidungsfindung. Das System verfügt über eine Wissensbasis, die die erforderlichen Daten für das Screening umfasst. Die Wissensbasis ist in Kriterien-Sätze organisiert, die in Kategorien wie Chirurgie oder Bildgebung unterteilt sind.

DE 10 2006 042 317 A1 bezieht sich auf ein Verfahren zur Übertragung digitaler Daten zwischen einer Mehrzahl von Modulen, denen jeweils mindestens eine Adresse zugeordnet ist. Der Zwischenspeicher für jedes Modul umfasst einen adressierbaren Datenspeicherbereich und eine Zustandskennung des Datenspeicherbereichs zur Information darüber, ob der Datenspeicherbereich mit neuen Daten beschreibbar ist oder gültige Daten zum Auslesen enthält.

EP 1850 342 A1 beschreibt eine Informationsaufnahmevorrictung. Die Informationsaufnahmevorrichtung fügt Daten einen Korrektur-Code hinzu und setzt ein Transfer-Flag.

WO 2006/044518 A2 betrifft ein Verfahren zur Bereitstellung von individuell angepassten, evidenzbasierte Interventionen Plänen, die auf die spezifischen Bedürfnisse einzelner Teilnehmer einer Gesamtbevölkerung abzielen.

US 2003/0060688 A1 bietet eine umfassende Lösung zur Verwaltung, die klinische Informationen aus unterschiedlichen Quellen sammelt, integriert und speichert. Das System findet Risikopersonen, bevor sie vermeidbar hohe Kostenerzeugen.

EP1 416 419 A2 beschreibt eine Technik zur Erzeugung Anonymität bei der Erhebung von Patientendaten, die Technik umfasst das Empfangen eines ärztlichen Gutachtens für einen Patienten, einschließlich Patienten-Identifikationsdaten. Eine Patientenakte wird nach einem anonymen Patienten-Identifier, der dem Patienten entspricht durchsucht.

WO 02/084531 A2 bezeiht sich auf eine Technik zum Anonymisieren von Einträgen in einer Eingabe-Datenquelle.

EP 1 471 454 beschreibt ein Patienten-Information-Management-System für die Prüfung eines Medikaments auf Nebenwirkungen mit bereits dem Patienten verschriebenen Medikamenten.

WO 2005/057465 A2 beschreibt eine Technik zur Prüfung von Nebenwirkungen einer Kombination von Medikamenten auch durch Personen ohne medizinische Kenntnisse.

WO 01/46016 A1 beschreibt die Bereitstellung einer Reihe von individuell angepassten Dosen nach einem zeitlichen Dosierungsschema.

### Übersicht über die Erfindung

Es ist die Aufgabe der vorliegenden Erfindung, den Datenaustausch mit einem Patientenverwaltungssystem, insbesondere die Dateneingabe in ein solches Patientenverwaltungssystem, zu verbessern.

Diese Aufgabe wird durch die Gegenstände der unabhängigen Ansprüchen gelöst.

Bevorzugte Ausführungsformen werden in den abhängigen Ansprüchen beschrieben.

Weitere bevorzugte Ausführungsformen sind:
1. Patientenverwaltungssystem, umfassend:
   ein Gerät (230), welches ein Patientenverwaltungssystem-Programm ausführt;
   eine Datenquelle (210) zum Bereitstellen medizinischer Daten; und
   eine Schnittstelleneinrichtung (100; 220), die zwischen dem Gerät (230) und der Datenquelle (210) eingebunden ist und Daten von der Datenquelle (210) empfängt, in Tastatur-Scan-Codes umwandelt, die für das Patientenverwaltungssystem-Programm spezifisch sind, und diese Tastatur-Scan-Codes an das Gerät (230) ausgibt.
2. Patientenverwaltungssystem gemäß Ausführungsform 1, wobei die Datenquelle (210) ein medizinisches Gerät ist.
3. Patientenverwaltungssystem gemäß Ausführungsform 2, wobei das medizinische Gerät ein Röntgen-Gerät oder ein Ultraschall-Untersuchungsgerät ist.
4. Patientenverwaltungssystem gemäß Ausführungsform 1, wobei die Datenquelle (210) eine grafische Eingabeschnittstelle ist.
5. Patientenverwaltungssystem gemäß einer der Ausführungsformen 1 bis 4, wobei die Schnittstelleneinrichtung (100) über eine Schnittstelle für den Transfer von medizinischen Daten mit der Datenquelle (210) verbunden ist.
6. Patientenverwaltungssystem gemäß Ausführungsform 5, wobei die Schnittstelle für den Transfer von medizinischen Daten eine GDT-Schnittstelle (105), eine BDT-Schnittstelle oder eine HL7-Schnittstelle ist.
7. Patientenverwaltungssystem gemäß einer der Ausführungsformen 1 bis 6, wobei die Schnittstelleneinrichtung mindestens einen Teil der von der Datenquelle (210) empfangenen Daten anhand von Regeln überprüft.
8. Patientenverwaltungssystem gemäß Ausführungsform 7, wobei die Prüfung des mindestens einen Teils der empfangenen Daten kategorienweise durchgeführt wird.
9. Patientenverwaltungssystem gemäß Ausführungsform 8, wobei die Kategorien Anamnesen, Befunde, Diagnosen, ICD Codierungen, Therapien, Abrechnungsziffern oder Formulare umfassen.
10. Patientenverwaltungssystem gemäß einer der Ausführungsformen 1 bis 9, wobei die Umwandlung in Tastatur-Scan-Codes anhand einer Zuordnungstabelle geschieht.
11. Patientenverwaltungssystem gemäß Ausführungsform 10, wobei die Zuordnungstabelle angibt, in welche Tastatur-Scan-Codes die von der Datenquelle (210) empfangenen Daten in Abhängigkeit von dem verwendeten Patientenverwaltungssystem-Programm umgewandelt werden.
12. Patientenverwaltungssystem gemäß einer der Ausführungsformen 1 bis 11, wobei das Ausgeben der Tastatur-Scan-Codes an das Gerät (230) kategorienweise ausgeführt wird.
13. Patientenverwaltungssystem gemäß Ausführungsform 12, wobei die Kategorien Anamnesen, Befunde, Diagnosen, ICD Codierungen, Therapien, Abrechnungsziffern oder Formulare umfassen.
14. Patientenverwaltungssystem gemäß einer der Ausführungsformen 1 bis 13, wobei das Ausgeben der Tastatur-Scan-Codes an das Gerät (230) das Injizieren der Tastatur-Scan-Codes in eine API des Geräts (230) umfasst.
15. Patientenverwaltungssystem gemäß einer der Ausführungsformen 1 bis 14, wobei die Tastatur-Scan-Codes in einem Pufferspeicher zwischengespeichert werden.
16. Patientenverwaltungssystem gemäß Ausführungsform 15, wobei bei dem Kategorienweisen Ausgeben der Tastatur-Scan-Codes die aktuell auszugebenden Tastatur-Scan-Codes in dem Pufferspeicher vor der Ausgabe mit einem Marker versehen werden, der anzeigt, dass die Ausgabe noch nicht abgeschlossen ist.
17. Patientenverwaltungssystem gemäß Ausführungsform 16, wobei die Ausgabe der Tastatur-Scan-Codes an das Gerät (230) wiederholt wird, wenn die Schnittstelleneinrichtung im Zusammenhang mit der Ausgabe der Tastatur-Scan-Codes and das Gerät (230) eine Fehlermeldung von dem Gerät (230) empfängt.
18. Patientenverwaltungssystem gemäß Ausführungsform 17, wobei die Wiederholung der Ausgabe der Tastatur-Scan-Codes an das Gerät (230) unter Verwendung der in dem Pufferspeicher zwischengespeicherten Tastatur-Scan-Codes geschieht.
19. Patientenverwaltungssystem gemäß einer der Ausführungsformen 16 bis 18, wobei nach der Ausgabe der Tastatur-Scan-Codes an das Gerät (230), wenn die Schnittstelleneinrichtung im Zusammenhang mit der Ausgabe der Tastatur-Scan-Codes and das Gerät (230) keine Fehlermeldung vom Gerät (230) empfängt, der Marker, der anzeigt, dass die Ausgabe noch nicht abgeschlossen ist, wieder gelöscht wird.
20.Patientenverwaltungssystem gemäß einer der Ausführungsformen 1 bis 19, wobei die Schnittstelleneinrichtung von dem Patientenverwaltungssystem-Programm einen Patienten-Score empfängt und diesen Score mit einem oder mehreren vorgegebenen Score-Werte vergleicht.
21. Patientenverwaltungssystem gemäß Ausführungsform 20, wobei die Schnittstelleneinrichtung eine Benachrichtigung auslöst, wenn der empfangene Patienten-Score einen der vorgegebenen Score-Werte übersteigt.
22. Patientenverwaltungssystem gemäß Ausführungsform 21, wobei die Benachrichtigung eine Behandlungsempfehlung oder eine Empfehlung zur Überweisung zum Facharzt oder Krankenhaus umfasst.
23. Patientenverwaltungssystem gemäß einer der Ausführungsformen 1 bis 22, wobei die Schnittstelleneinrichtung die vom Gerät (230) empfangenen Daten in einer Datenbank speichert.
24. Patientenverwaltungssystem gemäß Ausführungsform 23, wobei die Datenbank zur Speicherung der vom Gerät (230) empfangenen Daten eine externe Datenbank ist.
25. Patientenverwaltungssystem gemäß Ausführungsform 24, wobei die externe Datenbank auf einem Datenbankserver betrieben wird.
26. Patientenverwaltungssystem gemäß einer der Ausführungsformen 23 bis 25, wobei die Datenbank eine SQL-Datenbank ist.
27. Patientenverwaltungssystem gemäß einer der Ausführungsformen 1 bis 26, wobei die Schnittstelleneinrichtung eingerichtet ist, die vom Gerät (230) empfangenen Daten an ein weiteres Programm auszugeben.
28. Patientenverwaltungssystem gemäß Ausführungsform 27, wobei die Schnittstelleneinrichtung des Weiteren eingerichtet ist, die in der Datenbank gespeicherten Daten an das weitere Programm auszugeben.
29. Patientenverwaltungssystem gemäß einer der Ausführungsformen 27 und 28, wobei die Ausgabe der Daten anonymisiert erfolgt.
30. Patientenverwaltungssystem gemäß einer der Ausführungsformen 27 bis 29, wobei das weitere Programm eingerichtet ist, um in Erwiderung auf die von der Schnittstelleneinrichtung empfangenen Daten eine Behandlungsempfehlung für den/die Patienten auszugeben.
31. Patientenverwaltungssystem gemäß einer der Ausführungsformen 27 bis 30, wobei das weitere Programm eine Care-Engine ist.
32. Patientenverwaltungssystem gemäß einer der Ausführungsformen 1 bis 31, wobei die Schnittstelleneinrichtung eingerichtet ist, unter Verwendung einer Datenbank, die Wechselwirkungen zwischen Medikamenten beschreibt, die Daten in einer Kategorie auf mögliche Wechselwirkungen zu prüfen und eine Warnung auszugeben, wenn eine mögliche Wechselwirkung festgestellt wird.
33. Schnittstelleneinrichtung, die eingerichtet ist, um zwischen einem Gerät, welches ein Patientenverwaltungssystem-Programm ausführt, und einer Datenquelle zum Bereitstellen medizinischer Daten eingebunden zu werden, und die Daten von der Datenquelle empfängt, in Tastatur-Scan-Codes umwandelt, die für das Patientenverwaltungssystem-Programm spezifisch sind, und diese Tastatur-Scan-Codes an das Gerät ausgibt.
34. Schnittstelleneinrichtung gemäß Ausführungsform 34, wobei die Schnittstelleneinrichtung angepasst ist, um gemäß einer der Ausführungsformen 2 bis 32 betrieben zu werden.
35. Patientenverwaltungsverfahren zum Betreiben einer Schnittstelleneinrichtung, welche mit einem Gerät, welches ein Patientenverwaltungssystem-Programm ausführt, verbindbar ist, wobei das Verfahren die folgenden Schritte umfasst:
   Empfangen (320) von medizinischen Daten von einer Datenquelle zum Bereitstellen medizinischer Daten;
   Umwandeln (365) der medizinischen Daten in Tastatur-Scan-Codes, die für das Patientenverwaltungssystem-Programm spezifisch sind; und
   Ausgeben (370) der Tastatur-Scan-Codes an das Gerät.
36. Verfahren gemäß Ausführungsform 35, wobei die Datenquelle ein medizinisches Gerät ist.
37. Verfahren gemäß Ausführungsform 36, wobei das medizinische Gerät ein Röntgen-Gerät oder ein Ultraschall-Untersuchungsgerät ist.
38. Verfahren gemäß Ausführungsform 35, wobei die Datenquelle eine grafische Eingabeschnittstelle ist.
39. Verfahren gemäß einer der Ausführungsformen 35 bis 38, wobei die Schnittstelleneinrichtung über eine Schnittstelle für den Transfer von medizinischen Daten mit der Datenquelle verbunden ist.
40. Verfahren gemäß Ausführungsform 39, wobei die Schnittstelle für den Transfer von medizinischen Daten eine GDT-Schnittstelle, BDT-Schnittstelle oder HL7-Schnittstelle ist.
41. Verfahren gemäß einer der Ausführungsformen 35 bis 40, des Weiteren umfassend: Überprüfen (325; 330; 335, 340) von mindestens einem Teil der von der Datenquelle empfangenen Daten anhand von Regeln.
42. Verfahren gemäß Ausführungsform 41, wobei der Schritt des Überprüfens des mindestens einen Teils der empfangenen Daten kategorienweise durchgeführt wird.
43. Verfahren gemäß Ausführungsform 42, wobei die Kategorien Anamnesen, Befunde, Diagnosen, ICD Codierungen, Therapien, Abrechnungsziffern oder Formulare umfassen.
44. Verfahren gemäß einer der Ausführungsformen 35 bis 42, wobei der Schritt des Umwandelns der medizinischen Daten in Tastatur-Scan-Codes anhand einer Zuordnungstabelle geschieht.
45. Verfahren gemäß Ausführungsform 44, wobei die Zuordnungstabelle angibt, in welche Tastatur-Scan-Codes die von der Datenquelle empfangenen Daten in Abhängigkeit von dem verwendeten Patientenverwaltungssystem-Programm umgewandelt werden.
46. Verfahren gemäß einer der Ausführungsformen 35 bis 45, wobei das Ausgeben der Tastatur-Scan-Codes an das Gerät kategorienweise ausgeführt wird.
47. Verfahren gemäß Ausführungsform 46, wobei die Kategorien Anamnesen, Befunde, Diagnosen, ICD Codierungen, Therapien, Abrechnungsziffern oder Formulare umfassen.
48. Verfahren gemäß einer der Ausführungsformen 35 bis 47, wobei das Ausgeben der Tastatur-Scan-Codes an das Gerät das Injizieren der Tastatur-Scan-Codes in eine API des Geräts umfasst.
49. Verfahren gemäß einer der Ausführungsformen 35 bis 48, wobei die Tastatur-Scan-Codes in einem Pufferspeicher zwischengespeichert werden.
50. Verfahren gemäß einer der Ausführungsformen 46 bis 49, wobei der Schritt des kategorienweisen Ausgeben der Tastatur-Scan-Codes vor dem Schritt des Ausgebens umfasst: Markieren der aktuell auszugebenden Tastatur-Scan-Codes in dem Pufferspeicher mit einer Markierung, die anzeigt, dass die Ausgabe noch nicht abgeschlossen ist.
51. Verfahren gemäß einer der Ausführungsformen 35 bis 50, wobei der Schritt des Ausgebens der Tastatur-Scan-Codes an das Gerät wiederholt (390) wird, wenn die Schnittstelleneinrichtung im Zusammenhang mit der Ausgabe der Tastatur-Scan-Codes and das Gerät eine Fehlermeldung von dem Gerät empfängt.
52. Verfahren gemäß Ausführungsform 51, wobei die Wiederholung des Ausgebens der Tastatur-Scan-Codes an das Gerät unter Verwendung der in dem Pufferspeicher zwischengespeicherten Tastatur-Scan-Codes geschieht.
53. Verfahren gemäß einer der Ausführungsformen 50 bis 52, wobei das Verfahren nach dem Schritt des Ausgebens der Tastatur-Scan-Codes an das Gerät, den Schritt umfasst: Löschen der Markierung, die anzeigt, dass die Ausgabe noch nicht abgeschlossen ist, wenn die Schnittstelleneinrichtung im Zusammenhang mit der Ausgabe der Tastatur-Scan-Codes and das Gerät keine Fehlermeldung vom Gerät empfängt.
54. Verfahren gemäß einer der Ausführungsformen 35 bis 53, des Weiteren umfassend: Empfangen (345) eines Patienten-Score; und Vergleichen (350) des empfangenen Patienten-Score mit einem oder mehreren vorgegebenen Score-Werte.
55. Verfahren gemäß Ausführungsform 54, des Weiteren umfassend:
   Auslösen (355) einer Benachrichtigung, wenn der empfangene Patienten-Score einen der vorgegebenen Score-Werte übersteigt.
56. Verfahren gemäß Ausführungsform 55, wobei die Benachrichtigung eine Behandlungsempfehlung oder eine Empfehlung zur Überweisung zum Facharzt oder Krankenhaus umfasst.
57. Verfahren gemäß einer der Ausführungsformen 35 bis 56, des Weiteren umfassend: Speichern (360) die vom Gerät empfangenen Daten in einer Datenbank.
58. Verfahren gemäß Ausführungsform 57, wobei die Datenbank zur Speicherung der vom Gerät empfangenen Daten eine externe Datenbank ist.
59. Verfahren gemäß Ausführungsform 58, wobei die externe Datenbank auf einem Datenbankserver betrieben wird.
60. Verfahren gemäß einer der Ausführungsformen 57 bis 59, wobei die Datenbank eine SQL-Datenbank ist.
61. Verfahren gemäß einer der Ausführungsformen 35 bis 60, des Weiteren umfassend: Ausgeben der von dem Gerät empfangenen Daten an ein weiteres Programm.
62. Verfahren gemäß Ausführungsform 61, wobei der Schritt des Ausgebens der von dem Gerät empfangenen Daten an ein weiteres Programm die Ausgabe der in der Datenbank gespeicherten Daten an das weitere Programm umfasst.
63. Verfahren gemäß einer der Ausführungsformen 61 und 62, wobei das Ausgeben der Daten anonymisiert erfolgt.
64. Verfahren gemäß einer der Ausführungsformen 61 bis 63, wobei das weitere Programm eingerichtet ist, um in Erwiderung auf die von der Schnittstelleneinrichtung empfangenen Daten eine Behandlungsempfehlung für den/die Patienten auszugeben.
65. Verfahren gemäß einer der Ausführungsformen 61 bis 64, wobei das weitere Programm eine Care-Engine ist.
66. Verfahren gemäß einer der Ausführungsformen 35 bis 65, des Weiteren umfassend: Prüfen, unter Verwendung einer Datenbank, die Wechselwirkungen zwischen Medikamenten beschreibt, der Daten in einer Kategorie auf mögliche Wechselwirkungen; und Ausgeben einer Warnung, wenn eine mögliche Wechselwirkung festgestellt wird.

### Kurze Beschreibung der Figuren

- Fig. 1: zeigt eine schematische Übersicht des Aufbaus der Datenschnittstelle.
- Fig. 2: zeigt den schematischen Aufbau des erfindungsgemäßen Patientenver-waltungssystems.
- Fig. 3A und 3B: zeigen ein Ablaufdiagramm des erfindungsgemäßen Verfahrens.

### Detaillierte Beschreibung erfindungsgemäßer Ausgestaltungen

Wie zuvor beschrieben betrifft die Erfindung ein Patientenverwaftungssystem, bei dem eine Schnittstelleneinrichtung zwischen einem Gerät, welches ein Patientenverwaltungssystem-Programm ausführt, und einer Datenquelle zum Bereitstellen von medizinischen Daten eingebunden ist und die Daten von der Datenquelle empfängt, diese in für das Patientenverwaltungssystem-Programm spezifische Tastatur-Scan-Codes umwandelt, und diese Tastatur-Scan-Codes an das Gerät ausgibt.

Das Gerät, welches die Patientenverwaltungssystem-Software ausführt, kann ein handelsüblicher Personal Computer sein, denkbar ist es aber auch, dass wesentlich leistungsfahigere Servercomputer zum Einsatz kommen. Der Einsatz von leistungsfähigeren Servercomputern hat den Vorteil, dass diese einem erhöhten Datenaufkommen gerecht werden, in dem Fall, dass das Patientenverwaltungssystem-Programm in einem Krankenhaus, Klinikum oder einer sehr großen Arztpraxis zum Einsatz kommt.

Als Patientenverwaltungssystem-Programme können beispielsweise Programme wie ME-DISTAR oder PDE-TOP zum Einsatz kommen. Solche Computerprogramme, die auch unter dem Begriff Arztsoftware bekannt sind, unterstützen die Verwaltung, die Organisation und den Betrieb von Arztpraxen. Die Funktionalität solcher Arztsoftware umfasst die Patientenverwaltung, Karteiführung, Dokumenten-Management bis hin zur Abrechnung mit den Krankenkassen. Die Anbindung von elektronischen Geräten an diese Software ist möglich, beispielsweise um die Daten, die auf einer Versichertenkarte gespeichert sind, über ein Kartenlesegerät in diese Software einzugeben. Ebenso ist es möglich, Befunde elektronisch zu übermitteln.

Die Schnittstelleneinrichtung oder auch Datenschnittstelle DS ist zwischen dem Gerät, welches die Patientenverwaltungssoftware ausführt und einer Datenquelle eingebunden und empfängt Daten, insbesondere Patientendaten von der Datenquelle. In einer Ausführungsform können die Daten von der Datenquelle an die Schnittstelleneinrichtung über eine definierte GDT-Schnittstelle übertragen werden, dies hat den Vorteil, dass im Bereich der Medizininformatik solche Schnittstellen häufig anzutreffen sind und demnach sehr viele Datenquellen über diese Schnittstelle verfügen. Beispielsweise sind Ultraschallgeräte oder Röntgengeräte mit einer GDT-Schnittstelle ausgestattet, die einen schnellen und effizienten Datentransfer, zumindest der Grunddaten, ermöglicht. In der Regel werden im Fall eines Röntgengeräts die Patientengrunddaten über GDT übertragen, das Bilddaten über eine Schnittstelle gemäß dem Dicom- (Digital Imaging and Communications in Medicine) Standard gesendet werden. Des Weiteren können in manchen Ausführungsformen die medizinischen Daten über ein Eingabegerät, welches speziell zum Eingeben von medizinischen Daten eingerichtet ist, eingegeben werden. Solch ein Eingabegerät kann ein Personal Computer oder ein Laptop oder ein Tablet-PC sein, welcher über eine grafische Eingabeschnittstelle verfügt, was den Vorteil hat, dass über eine grafische Eingabeschnittstelle die Eingabe zeitsparend erfolgen kann, da die auf einer grafischen Schnittstelle vorhandenen grafischen Buttons leicht erkennbar und anklickbar sind, was die Eingabegeschwindigkeit erhöht. Insbesondere, wenn das Eingabegerät mit einem TouchScreen ausgestattet ist, kann die Eingabe nicht nur mittels Maus und Tastatur erfolgen, sondern durch die Verwendung des Fingers oder eines hierfür vorgesehenen Eingabestiftes, was eine noch schnellere und intuitivere Bedienung der Eingabe ermöglicht.

Die derart eingegebenen Daten werden, wie zuvor beschrieben, über eine hierfür vorgesehen Schnittstelle an die Schnittstelleneinrichtung übergeben. Die Schnittstelleneinrichtung wandelt diese Daten in Tastatur-Scan-Codes um, welche an das angeschlossene Gerät ausgegeben werden, um in das Patientenverwaltungssystem-Programm eingegeben zu werden. Tastatur-Scan-Codes sind in der Computertechnik Daten, die von der Tastatur eines Rechners an diesen gesendet wird, wenn eine Taste gedrückt oder losgelassen wird. Die zu verwendenden Tastatur-Scan-Codes sind in der Regel abhängig von der eingesetzten Patientenverwaltungssystem-Software. Der Schnittstelleneinrichtung wird die verwendete Software mitgeteilt, und die Schnittstelleneinrichtung kann aus einer Tabelle ermitteln, welcher Satz von Tastatur-Scan-Codes für diese Software benötigt werden, und kann diese dann entsprechend verwenden. Dies hat den Vorteil, dass die Schnittstelleneinrichtung schnell auf eine geänderte Patientenverwaltungssystem-Software angepasst werden kann. Dies hat darüber hinaus den weiteren Vorteil, dass die Eingangsschnittstelle der Schnittstelleneinrichtung, nämlich die zuvor beschriebene GDT-Schnittstelle, davon unberührt bleibt, wenn sich die verwendete Patientenverwaltungssystem-Software ändert. Dies ermöglicht es dem Anwender, die Eingabe in das Patientenverwaltungssystem in gewohnter Weise auszuführen, im idealen Fall bleibt es für den Verwender des Patientenverwaltungssystems unmerkbar, welche Patientenverwaltungssystem-Software verwendet wird. Deshalb fallen bei einem Wechsel des Patientenverwaltungssystem-Programms keine Umschulungen an, um mit dem neuen Programm umgehen zu können.

Die Funktionalität der Schnittstelleneinrichtung kann in manchen Ausführungsformen in einer betriebssystemunabhängigen Programmiersprache, beispielsweise TCL, programmiert sein, so dass die Schnittstelleneinrichtung unabhängig von dem gewählten Betriebssystem ist, was insbesondere dann von Vorteil ist, wenn in einer Praxis unterschiedliche Betriebssysteme, wie Windows, Linux und Mac OS zum Einsatz kommen. Durch die Unabhängigkeit der Datenschnittstelle vom gewählten Betriebssystem ist dann die Bedienung und das Look-and-Feel, d.h. das Benutzerempfinden, immer das gleiche, was Eingewöhnungsphasen zur Bedienung der Software wegfallen lässt.

Die erfindungsgemäße Schnittstelleneinrichtung ist unabhängig von Datenstrukturänderungen, die sich bei Aktualisierungen/Updates der Endbenutzer-Software ergeben können. Würde die Kommunikation zwischen der Schnittstelleneinrichtung und dem Patientenverwaltungssystem-Programm mittels einer vordefinierten Schnittstelle, wie beispielsweise der GDT-Schnittstelle, erfolgen, würde sich in der Regel bei einem Programm-Update die Eingangsschnittstelle des Patientenverwaltungssystems ändern und eine Aktualisierung der Schnittstelleneinrichtung wäre notwendig. Da in der Regel der Anbieter des Patientenverwaltungssystem-Programms die Datenstruktur der Eingangsschnittstelle nicht offenlegt, um es der Konkurrenz zu erschweren, sich auf eine geänderte Datenstruktur anzupassen, müsste eine geänderte Datenstruktur der Geräteschnittstelle beispielsweise durch ein sogenanntes Reverse-Engineering rekonstruiert werden, was einen sehr großen zeitlichen Aufwand mit sich bringt. Da in der Regel aber, die grafische Eingabemaske des Patientenverwaltungssystem-Programms sich nicht ändert, um eine Kontinuität in der Bedienung des Programms zu sichern, hat die Verbindung von Tastatur-Scan-Codes zur Übermittlung der Eingabe in das PVS-Programm den Vorteil, dass man gerade diese Konstanz der Strukturierung der grafischen Eingabemaske ausnutzen kann.

Die Schnittstelleneinrichtung weist in manchen Ausführungsformen noch eine gewisse Eigenintelligenz auf, d.h. die Datenschnittstelle ist in der Lage, die unterschiedlichen empfangenen Daten einzuordnen und in der Endbenutzer-Software richtig einzutragen. Hierzu ist die Schnittstelleneinrichtung in der Lage, die empfangenen Daten in Grundkategorien einzuordnen, wobei die Grundkategorien Anamnesen, Befunde, Diagnosen, ICD-Codes, Therapien, Prozedere, Abrechnungsziffern und Formulare umfassen können. Die Aufzählung der Grundkategorien ist nicht abschließend und kann noch beliebig erweitert werden. Dies hat den Vorteil, dass eine dynamische Anpassung an die jeweiligen Ansprüche des Benutzers möglich ist. Beispielsweise hat ein Facharzt für Orthopädie andere Ansprüche an die verwendeten Kategorien als ein Kardiologe oder ein Hautarzt.

Um die Kategorie der jeweiligen Untermenge der von der Datenquelle bereitgestellten medizinischen Daten zu ermitteln, greift in manchen Ausführungsformen die erfindungsgemäße Schnittstelleneinrichtung auf Information zurück, die zusammen mit den Daten übertragen werden. Beispielsweise kann die Grundkategorie durch einen den eigentlichen Patientendaten vorangestaltenen Präfix definiert sein. Beispielsweise kann ein Blutdruckwert, der von der Datenquelle bereitgestellt wird, durch einen Präfix "RR" gekennzeichnet sein. Das Kürzel "RR" ist lediglich als beispielhaft anzusehen. Es ist üblich, Blutdruckwerte derart anzugeben, so dass zuerst der systolische und dann der diastolische Druckwert angegeben wird. Der systolische Wert entspricht dem Gefäß-Innendruck beim Ausdehnen des Gefäßes, der diastolische Wert entspricht dem Gefäß-Innendruck beim Zusammensziehen, weshalb der systolische immer größer ist als der diastolische Wert. Sind die gemessenen Werte beispielsweise 140 und 90, können diese über die Datenschnittstelle übergeben werden unter Verwendung der Zeichenfolge "RR 140/90". "RR" ist die übliche Abkürzung für Riva-Rocci, ein italienischer Arzt, der erstmalig die Armmanschette zur Messung des Blutdrucks beschrieben hat und zu dessen Andenken die Abkürzung "RR" eingeführt wurde. Alternativ könnte auch die Abkürzung "BD" für "Blutdruck" oder "BP" für "Blood Pressure" verwendet werden. Stellt die Schnittstelleneinrichtung fest, dass eine Zeichenkette bereitgestellt wird, die mit "RR" beginnt, kann diese somit als Blutdruckwert erkannt werden. Da dem Bediener des PVS-Programms bekannt ist, in welchem Teil der Eingabemaske Blutdruckwerte eingegeben werden sollen, ist es möglich, durch den Einsatz der Tastatur-Scan-Codes das richtige Eingabefeld anzusteuern und dort die Blutdruckwerte einzugeben.

Das Ermitteln der mit den bereitgestellten Daten verbundenen Kategorie ermöglicht es der Schnittstelleneinrichtung, diese Daten zu überprüfen. Beispielsweise kann, emeut unter Verwendung des vorher beschriebenen Blutdruckwertes, die Datenschnittstelle überprüfen, ob der erste Wert, der dem systolischen Blutdruckwert entspricht, größer ist als der nachfolgende diastolische Wert. Ist dies nicht der Fall, ist davon auszugehen, dass die bereitgestellten Daten fehlerhaft sind, und es ist dann möglich, an den Benutzer des PVS-Systems eine entsprechende Wammeldung auszugeben. Dies hat den Vorteil, dass Fehleingaben zeitnah entdeckt werden können, in der Regel unmittelbar nach der Untersuchung des Patienten. Fiele eine Dateninkonsistenz zwischen der Diagnose und den Abrechnungsziffern erst beispielsweise bei der Abrechnung mit der Krankenkasse auf, was einige Tage oder Wochen nach der Untersuchung des Patienten sein kann, ist es in der Regel nicht mehr möglich, diesen Fehler zu korrigieren.

Des Weiteren ist es der Schnittstelleneinrichtung möglich, die Plausibilität der Daten anhand eines Regelwerks zu überprüfen.

Bei einer solchen Plausibilitätsprüfung überprüft die Schnittstelleneinrichtung die Daten anhand eines Regelwerks, bevor diese Daten an die Endbenutzer-Software weitergeleitet werden, beispielsweise wird überprüft, ob die erhobenen ICD-Codes der Diagnose zu den Medikamenten passen oder ob die Seitenlokalisationen in den Diagnosen (z.B. rechtes oder linkes Knie) zu den Seitenlokalisationen der bei den Ziffern gewählten OPS-Schlüsseln passt. Dies hat den Vorteil, dass eine derartige Inkonsistenz innerhalb des Datensatzes rechtzeitig entdeckt werden kann, insbesondere im Hinblick darauf, dass so ärztliche Kunstfehler vermieden werden können. Wurde beispielsweise eine Diagnose aufgrund eines Röntgenbilds von dem rechten Knie angefertigt, erfolgt aber eine spätere Operationsplanung für das linke Knie, kann das erfindungsgemäße Patientenverwaltungssystem rechtzeitig eine entsprechende Warnung oder Fehlermeldung ausgeben.

Des Weiteren bietet sich der Vorteil, dass es bei dem erfindungsgemäßen Patientenverwaltungssystem nicht notwendig ist, die eigentliche Schnittstelle zur Dateneingabe des PVS-Programms zu nutzen. Diese Schnittstelle des PVS-Programms ist in der Regel eine grafische Eingabemaske, die eine Velzahl von Eingabefeldern aufweist und in die der Benutzer die Patienteninformation eingeben kann. Der Großteil der derzeit verfügbaren PVS-Programme ist werbungsfinanziert, d.h. dass während der Eingabe der Daten in die Programmeingabeschnittstelle im Grunde unerwünschte Werbefenster geöffnet werden oder andere Werbeeinblendungen erfolgen. In bestimmten Fällen, z.B., wenn bei dem Patienten eine Migräne-Erkrankung diagnostiziert wurde, könnte eine Werbeeinblendung erfolgen, die dem Arzt ein bestimmtes Migräne-Medikament empfiehlt.

Da bei dem efindungsgemäßen PVS-System die Eingabe der medizinischen Daten in manchen Ausführungsformen über eine weitere Datenquelle erfolgt, umgeht man so die werbungsbehaftete Schnittstelle. Jedoch hat eine Werbeeinblendung in Form eine zusätzlich sich öffnenden Fensters zur Folge, dass sich der Eingabefokus ändert, d.h. nach dem Öffnen des Werbefensters ist der Eingangsfokus nicht mehr das zuvor angesteuerte Eingabefeld der Schnittstelle, sondern ist das Werbungsfenster mit einer entsprechenden Taste zum Quittieren dieses Werbefensters. Deshalb ist in manchen Ausführungsformen die erfindungsgemäße Datenschnittstelle oder Schnittstelleneinrichtung eingerichtet, um einen solchen Fokuswechsel festzustellen und entsprechend darauf zu reagieren. Eine solche entsprechende Reaktion auf eine Werbeeinblendung könnte beispielsweise das Quittieren des Werbefensters und danach Rücksetzen des Eingabefokus in das zuletzt besuchte Eingabefeld umfassen. Des Weiteren führt nicht nur das Öffnen des Werbefensters zu einer ungewollten Fokusanderung, sondern auch vom Betriebssystem erzeugte Fehlermeldungen oder Benachrichtigungen. Öffnet sich beispielsweise ein Benachrichtigungsfenster, das den Benutzer dazu auffordert, die Festplatte aufzuräumen, was unter den Windows-Betriebssystemen üblicherweise dann passiert, wenn die Kapazität der Festplatte weitgehend erschöpft ist, muss dieses Fenster ebenso quittiert werden und der Eingabefokus zurückgesetzt werden. Solche Systemfehlermeldungen können über die entsprechende Programmierschnittstelle (application programming interface - API) abgefragt werden.

In manchen Ausführungsformen ist die Schnittstelleneinrichtung eingerichtet, um vom dem Gerät abzufragen, an welcher Stelle in dem aktuellen Fokus-Fenster zur Zeit die Eingabe erfolgen kann. Das aktuelle Fokus-Fenster verfügt in der Regel über mehrere Eingabefelder, in die beispielsweise der Patienten-Name, -Vorname sowie dessen gemessener Blutdruck eingegeben werden kann. Jedes dieser Eingabefelder verfügt zur Kennzeichnung über eine einzigartige ID, und eben dies ID kann abgefragt werden, um zu bestimmen, welches Eingabefeld aktuell aktiv ist. Wurde beispielsweise bei der Eingabe der Patientengrunddaten eine Fehlermeldung erzeugt und die Eingabe somit unterbrochen, ist nicht direkt nachvollziehbar bei welchem Eingabefeld die Unterbrechung aufgetreten ist. Durch Abfrage der ID des Eingabefelds kann bestimmt werden, wo die Eingabe fortgesetzt werden kann. Dies hat den Vorteil, dass somit nicht die Eingabemaske zurückgesetzt werden muss um diese in einen definierten Zustand zu versetzen um dann die Daten noch einmal komplett zu senden, sondern dass die Eingabe der Daten unter Reduzierung der erforderlichen Datenübertragungsbandbreite fortgesetzt werden kann. Darüber hinaus kann das Eingabefeld auch inhaltlich geprüft werden, so kann zum Beispiel das Eingabefeld, in welches das Datum einer Untersuchung eingefügt wird, darauf geprüft werden, ob das Datum dem aktuellen Datum oder dem Datum der Untersuchung entspricht.

Darüber hinaus ist die Schnittstelleneinrichtung in bestimmten Ausführungsformen dazu eingerichtet, die übergebenen Daten in eine beliebige, modular ansteuerbare Datenbank abzuspeichern. Dies hat den Vorteil, dass, wenn es nach einer der zuvor diskutierten Abbrüche oder Fehlermeldungen notwendig ist, die Daten noch einmal zu versenden, kann auf diese Daten zurückgegriffen werden. Des Weiteren können durch die Speicherung der Patientendaten eventuelle Unverträglichkeiten zwischen den aktuell verordneten Medikamenten und zuvor verordneten Medikamenten berücksichtigt werden. Wird beispielsweise festgestellt, dass dem Patient zuvor ein Blutverdünnungsmittel verschrieben wurde, und wurde dem gleichen Patienten aktuell ein Schmerzmittel verschrieben, das ebenso eine Blutverdünnung bewirken kann, kann eine entsprechende Warnung ausgegeben werden, dass sich die Wirkungen dieser beiden Medikamente verstärken. In anderen Fällen könnte berücksichtigt werden, dass die Wirksamkeit mancher Antibiotika die Wirksamkeit von manchen Kontrazeptiva unerwünscht beeinflusst, und eine entsprechende Warnung kann ausgegeben werden. Als eine solche Datenbank kann in manchen Ausführungsformen eine SQL-Datenbank zum Einsatz kommen.

Im Folgenden werden bevorzugte Ausgestaltungen noch einmal unter besonderer Berücksichtigung der beigefügten Zeichnungen erläutert.

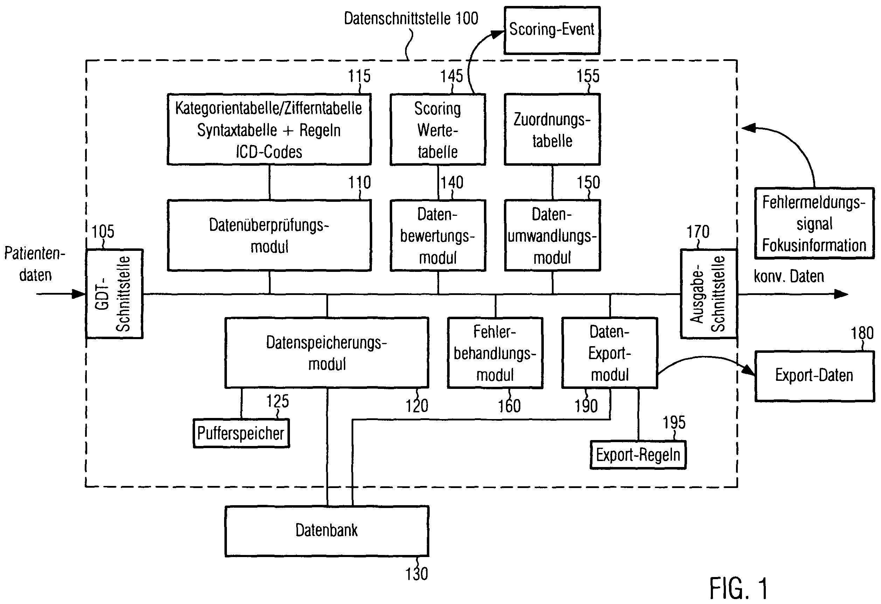

Fig. 1 zeigt eine Ausführungsform der erfindungsgemäßen Datenschnittstelle 100. Die Datenschnittstelle empfängt medizinische Daten über ein hierfür vorgesehene Schnittstelle 105, welche in bestimmten Ausführungsformen eine GDT-Schnittstelle ist. Des Weiteren umfasst die Datenschnittstelle ein Datenüberprüfungsmodul 110. Dieses Datenüberprüfungsmodul ist eingerichtet, um die empfangenen medizinischen Daten kategorienweise zu überprüfen. Hierbei findet eine Überprüfung bezüglich der Syntax statt. Entspricht der Aufbau der empfangenen Daten nicht der erwarteten Syntax, kann eine entsprechende Fehlermeldung ausgegeben werden und der Benutzer kann die Eingabe korrigieren. In anderen Ausführungsbeispielen kann das Datenüberprüfungsmodul Syntaxfehler selbständig korrigieren, beispielsweise wird ein Blutdruckwert "RR 90/140" empfangen, aber die anzuwendende Syntax verfangt, dass der erste Wert der größere ist, kann das Datenüberprüfungsmodul die beiden Zahlenwerte austauschen. Das Datenüberprüfungsmodul bezieht die Information betreffend die Syntax und weitere Regeln von einer Tabelle 115. In dieser Tabelle sind zu erwartende Kategorien und Ziffern abgelegt und damit verknüpfte Regeln, sowie die zugrundeliegende Syntax sind in Tabellen abgelegt. Ebenso befinden sich ICD-Codes in der Tabelle 115.

Die Schnittstelleneinrichtung umfasst des Weiteren ein Datenspeicherungsmodul 120. Dieses Datenspeicherungsmodul speichert die empfangenen Patientendaten in einer hierfür vorgesehenen Speichereinheit ab. In bestimmten Ausführungsformen ist diese hierfür vorgesehene Speichereinheit eine Datenbank 130. Diese Datenbank kann von der Schnittstelleneinrichtung getrennt sein, beispielsweise kann diese sich auf einen Datenbankserver befinden. Insbesondere kann in bestimmten Ausführungsformen diese Datenbank eine SQL-Datenbank sein, welche für diese Zwecke geeignet ist.

Die Schnittstelleneinrichtung der Fig. 1 zeigt des Weiteren ein Datenbewertungsmodul, das in bestimmten Ausführungsformen in der Schnittstelleneinrichtung ausgeführt ist. Dieses optionale Datenbewertungsmodul 140 ist in der Lage, anhand einer Wertetabelle 145 und unter Berücksichtigung der vorhandenen Patientendaten ein Patienten-Score zu bilden. Dieser Patienten-Score stellt ein Maß dafür dar, wie wahrscheinlich eine bestimmte Erkrankung des Patienten ist. Beispielsweise fließt in den Patienten-Score das Alter, das Geschlecht, das Körpergewicht, der Blutdruckverlauf über einen bestimmten Zeitraum, bestehende Diagnosen sowie aktuelle Befunde ein. Alle diese Faktoren werden mit Gewichtungswerten, welche empirisch ermittelt sein können, versehen, und diese Gewichtungsfaktoren werden aufsummiert. Dieser Patienten-Score kann mit einem oder mehreren vordefinierten Werten verglichen werden. Liegt der Patienten-Score beispielsweise über einem ersten Grenzwert, kann eine Nachricht erzeugt werden, die empfiehlt, diesen Patienten ein bestimmtes Medikament zu verschreiben oder eine bestimmte Therapie anzuraten. Liegt der Patienten-Score über einem weiteren, größeren Wert, kann empfohlen werden, den Patienten direkt in ein Krankenhaus zu überweisen. In manchen Ausführungsformen wird der Patienten-Score nicht von der Schnittstelleneinrichtung gebildet, sondern kann von außen, beispielsweise von der zuvor erwähnten Care-Engine, in die Schnittstelleneinrichtung eingegeben werden.

Bestimmte Ausführungsformen der Schnittstelleneinrichtung weisen ein Fehlerbehandlungsmodul 160 auf. Dieses Fehlerbehandlungsmodul fragt entsprechende Programmierschnittstellen des Betriebssystems ab, ob eine Fehlermeldung erzeugt wurde. Wenn eine solche Fehlermeldung erzeugt wurde, kann abgeklärt werden, ob das PVS-Programm die Fehlermeldung erzeugt hat, oder ob die Fehlermeldung von dem Betriebssystem erzeugt wurde. Handelt es sich um eine von dem Betriebssystem erzeugte Fehlermeldung, kann diese dem Benutzer angezeigt werden, damit dieser den betriebssystemspezifischen Fehler entsprechend behandeln kann. Ist jedoch die Fehlermeldung von dem PVS-Programm erzeugt worden, bedarf es einer weiteren Analyse durch die Schnittstelleneinrichtung, denn eine Fehlermeldung des PVS-Programms kann beispielsweise durch eine unvollständige oder fehlerhafte Dateneingabe hervorgerufen sein. In diesem Fall wird der zusammen mit der Fehlermeldung ausgegebene Fehlercode ausgewertet. Zeigt der Fehlercode an, dass die an das PVS-Programm übergebene Daten nicht den erwarteten Daten entsprechen, können sämtliche Daten, die zu der derzeitig übertragenen Kategorie gehören, erneut versendet werden.

Hierzu greift die Schnittstelleneinrichtung auf einen Pufferspeicher 125 zurück. Dieser Pufferspeicher ist mit dem Datenspeicherungsmodul 120 verbunden. In diesem Pufferspeicher können die zur Ausgabe bestimmten Daten in ihrer Gesamtheit gespeichert sein, können aber auch kategorienweise gespeichert sein, oder es kann sich lediglich eine Kategorie der Daten in dem Pufferspeicher befinden. Dies hat den Vorteil, dass eine erneute manuelle Eingabe der Daten nicht erforderlich ist, sondern es kann auch die in den Pufferspeicher befindenden Daten zurückgegriffen werden.

Des Weiteren umfasst die Datenschnittstelle ein Datenumwandlungsmodul 150. Dieses Datenumwandlungsmodul wandelt die empfangenen medizinischen Daten in Tastatur-Scan-Codes um. Hierzu wird anhand der zu der jeweiligen Daten zugehörigen Kategorie festgestellt, in welchen Teil der Eingabeschnittstelle des PVS-Programms diese Daten eingegeben werden müssen, und entsprechende Steuercodes werden erzeugt. Beispielsweise ist es möglich, zwischen den einzelnen Eingabebereichen mittels einer Tabulatortaste zu wechseln, deshalb können beispielsweise ein oder mehrere Tastatur-Scan-Codes, die der Eingabe einer Tabulatortaste entsprechen, eingefügt werden. Diese Angaben können einer Zuordnungstabelle 155 entnommen werden.

Diese Zuordnungstabelle oder Tastatur-Scan-Code-Tabelle weist auszugebenden Zeichen einen Tastatur-Scan-Code zu, das heißt dass Zeichen und auch Steuerzeichen unter Verwendung der Tastatur-Scan-Codes kodiert ausgegeben werden. Darüber hinaus weist die Tastatur-Scan-Code-Tabelle in manchen Ausgestaltungen noch die zuvor besprochenen einzigartigen IDs auf, die zur Kennzeichnung und Abfrage der Eingabefelder dienen. Diese IDs sind in der Tastatur-Scan-Code-Tabelle mit den jeweiligen, zugehörigen Kategorien verknüpft. Dies hat den Vorteil, dass die IDs der Eingabefelder, die bei der Übertragung der Daten einer bestimmten Kategorie angesteuert werden sollen, unmittelbar zur Verfügung stehen.

Das Datenumwandlungsmodul wandelt die empfangenen medizinischen Daten in bestimmten Ausführungsformen kategorieweise in Tastatur-Scan-Codes um, dies hat den Vorteil, dass, wenn die Datenübertragung fehlschlägt, muss lediglich die zuletzt übertragene Kategorie erneut übertragen werden, wodurch Übertragungsbandbreite eingespart werden kann. Die kategorieweise erzeugten Tastatur-Scan-Codes werden in bestimmten Ausführungsformen kategorieweise in den Pufferspeicher 125 abgelegt.

Die erfindungsgemäße Schnittstelleneinrichtung umfasst in bestimmten Ausführungsformen des Weiteren ein Datenexportmodul 190, welches dazu eingerichtet ist, die in der Datenbank 130 abgelegten Daten aus dieser Datenbank zu extrahieren, diese Daten in Gänze oder teilweise für den Export vorzubereiten und diese Daten dann in Form von Exportdaten 180 auszugeben. Die Aufarbeitung der Daten aus der Datenbank erfolgt unter Einhaltung bestimmter Exportregeln 195. Beispielsweise kann in den Exportregeln festgelegt sein, dass die Daten nur anullisiert ausgegeben werden dürfen, in diesem Fall werden die Daten ohne Angaben von Patientennamen ausgegeben, und anstelle des Patientennamens könnte beispielsweise lediglich eine willkürlich gewählte Nummer ausgegeben werden, anhand derer es nicht möglich ist, auf den jeweiligen Patienten zurückzuschließen. Solch exportierte Daten könnten an eine entsprechende API des Gerätes, das das Patientenverwaltungssystem-Programm ausführt, weitergegeben werden, so dass dieses Gerät dann diese Daten per eMail an eine sogenannte Care Engine versendet.

Eine solche Care Engine dient dazu, anhand der Patientendaten eine Behandlungsempfehlung auszusprechen, um es dem behandelnden Arzt zu erteichtern, einen Behandlungsplan auszuarbeiten. Insbesondere im Falle von multimorbiden Patienten bedeutet es für den behandelnden Arzt eine deutliche Mehrbelastung, alle Wechselwirkungen der für die einzelnen Erkrankungen zu verschreibenden Medikamente in ihrer Gesamtheit zu berücksichtigen. Diese Arbeit kann zeitsparend von einer hierfür eingerichteten Care Engine übernommen werden, die dann ihrerseits eine Behandlungsempfehlung ausgibt. Wie zuvor beschrieben, kann eine Care-Engine dazu eingerichtet sein, um alternativ oder zusätzlich zur Behandlungsempfehlung einen Patienten-Score an die Schnittstelleneinrichtung zu senden.

Die Exportdaten 180 können in bestimmten Ausführungsformen anstatt an eine Care Engine an ein System zur Versorgungsforschung versendet werden. Solche Versorgungsforschungssysteme benutzen anonymisierte Daten, um die Wirksamkeit von unterschiedlichen Medikamenten anhand einer großen Gesamtheit von Patienten statisch zu untersuchen. Nimmt ein Arzt an solch einem Programm zur Versorgungsforschung teil, ist dies mit einer Doppelerfassung der Daten verbunden, da die Patientendaten einerseits in ein PVS-Programm eingepflegt werden müssen, es aber keine praktikable Möglichkeit gibt, diese Daten wieder aus dem PVS-Programm zu extrahieren. Deshalb müssen die Patientendaten doppelt eingegeben werden, so dass diese nicht nur in das PVS-Programm, sondern auch noch in ein Programm zur Versorgungsforschung eingegeben werden. Diese doppelte Dateneingabe schreckt in der Regel von der Teilnahme an einem solchen Versorgungsforschungsprogramm ab. Die erfindungsgemäße Schnittstelleneinrichtung bietet in bestimmten Ausführungsformen den Vorteil, dass eine solche doppelte Datenerfassung nicht mehr notwendig ist, und bietet darüber hinaus den Vorteil, dass über das optionale Datenexportmodul ein solcher Datenexport ohne merklichen Mehraufwand bewerkstelligt werden kann.

Die Datenschnittstelle verfügt über eine Ausgabeschnittstelle 170, welche die in Tastatur-Scan-Codes konvertierte Daten an das Gerät, welches das PVS-Programm ausführt, ausgibt. Bei der Ausgabe dieser Daten werden die erzeugten Tastatur-Scan-Codes in eine API des Zielrechners injiziert, dies bedeutet, dass der Zielrechner die injizierten Daten als Tastenanschläge interpretiert.

Des Weiteren ist das Fehlerbehandlungsmodul in der Lage, die Datenerfassung des PVS-Programms in einen definierten Zustand zurückzusetzen. Das heißt, es werden die entsprechenden Tastatur-Scan-Codes, d.h. Tastaturbefehle, an die Endbenutzer-Software geschickt, die das PVS-Programm veranlassen, die Eingabeschnittstelle in einen definierten Ausgangszustand zurückzusetzen, von dem aus die Dateneingabe erfolgen kann.

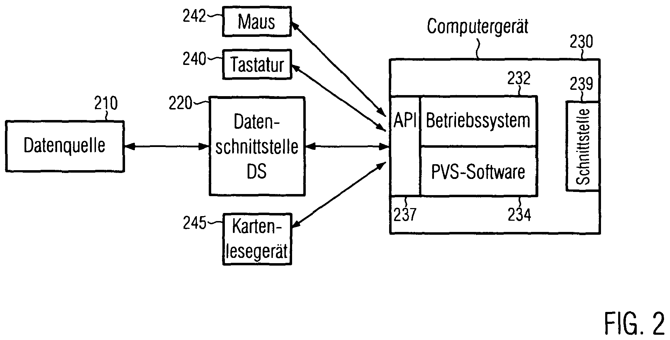

Fig. 2 zeigt eine schematische Übersicht über das erfindungsgemäße Patientenverwaltungssystem. Das Computergerät 230, d.h. das Gerät, welches die PVS-Software ausführt, umfasst ein Betriebssystem 232, die PVS-Software 234 sowie eine Programmierschnittstelle API 237. Das System umfasst des Weiteren die Schnittstelleneinrichtung oder Datenschnittstelle DS 220 sowie die Datenquelle 210. Die Datenquelle ist mit der Schnittstelleneinrichtung 220 kommunikativ verbunden, so dass die Schnittstelleneinrichtung von der Datenquelle Daten empfangen kann, und so dass in bestimmten Ausführungsformen die Datenquelle Fehlermeldungen, die von der Schnittstelleneinrichtung erzeugt oder weitergegeben werden, empfangen kann. Die Datenquelle kann beispielsweise ein medizinisches Gerät mit einer GDT-Schnittstelle sein oder auch eine grafische Eingabeeinrichtung, wie beispielsweise ein medizinischer Tablet-PC. Die Eingabeschnittstelle behandelt die von der Datenquelle bereitgestellten Daten, wie zuvor in Zusammenhang mit Fig. 1 beschrieben. Danach gibt die Eingabeschnittstelle die Daten an, die Programmierschnittstelle 237 des Computergeräts 230 aus. Das Computergerät 230 kann des Weiteren verbunden sein mit einer Tastatur 240, einer Maus 242 und einem Kartenlesegerät 245. In manchen Ausführungsformen ist das Kartenlesegerät 245 in der Tastatur 240 integriert. In bestimmten Ausführungsformen weist das Computergerät eine weiter Schnittstelle 239 auf, die es dem Computergerät ermöglicht, mit anderen Computern zu kommunizieren. Beispielsweise kann die Schnittstelle 239 eine Netzwerkschnittstelle sein, welche es ermöglicht, dass Daten über Kommunikationsprotokolle, wie beispielsweise TCP/IP, übertragen werden. Das Computergerät kann über diese Netzwerkschnittstelle mit dem zuvor erwähnten Versäumungsforschungsserver verbunden sein, oder aber auch mit einer Care Engine oder im einfachsten Fall mit weiteren Computergeräten, die Bestandteil eines Computemetzwerkes beispielsweise in einer Praxis oder einem Krankenhaus sind.

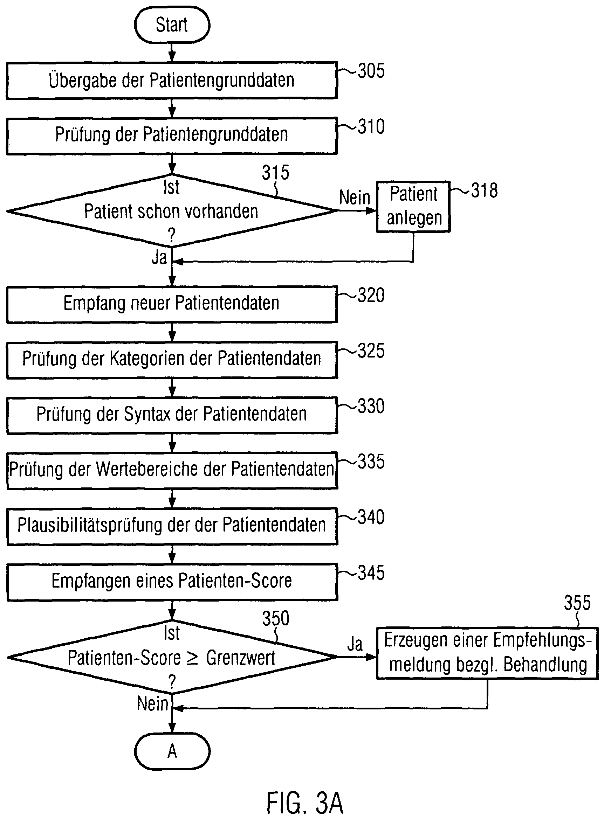

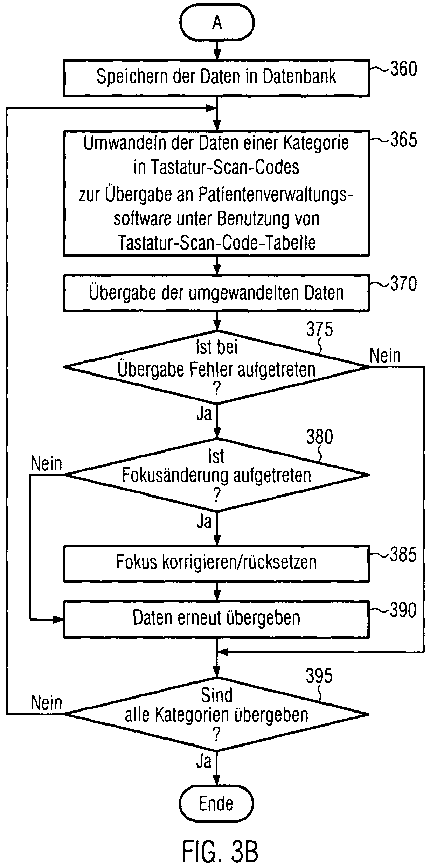

Fig. 3 zeigt schematisch ein Verfahren, nach dem Ausführungsformen des erfindungsgemäßen Patientenverwaltungssystems betrieben werden kann. Wie aus der vorherigen Beschreibung klar, sind nicht alle Schritte des beschriebenen Verfahrens efindungswesentlich und sind daher als optional anzusehen.

In Schritt 305 erfolgt die Übergabe von Patientengrunddaten an die Eingabeschnittstelle. In Schritt 310 werden diese Patientengrunddaten überprüft, beispielsweise darauf, ob der Patient schon vorhanden ist in Schritt 315. Ist der Patient noch nicht vorhanden, wird dieser in Schritt 318 neu angelegt. Danach werden in Schritt 320 neue Patientendaten empfangen. Dies geschieht beispielsweise über die zuvor diskutierte GDT-Schnittstelle. In Schritt 325 werden die Kategorien der empfangenen Daten geprüft, beispielsweise anhand einer Kategorientabelle oder einer Ziffemtabelle.

Danach wird in Schritt 330 die Syntax der empfangenen Daten überprüft, beispielsweise anhand einer ICD-Codetabelle.

In Schritt 335 werden in bestimmten Ausführungsformen die Wertebereiche der empfangenen Daten anhand einer Wertebereichstabelle überprüft, und eventuell korrigiert. In Schritt 340 erfolgt eine Plausibilitätsprüfung der empfangenen Daten anhand einer Vergleichs der ICD-Codes, der OPS-Schlüssel, Ziffern oder Medikamentenlisten. Beispielsweise kann hier ein Vergleich von Seitenlokalisierungen in den ICD-Codes und den Abrechnungsziffem durchgeführt werden.

In Schritt 345 wird ein Patienten-Score ermittelt, in Schritt 350 wird dieser Patienten-Score mit einem Grenzwert verglichen. Obwohl hier nur der Vergleich mit einem Grenzwert gezeigt ist, ist in manchen Ausführungsformen auch der Vergleich mit mehreren Grenzwerten möglich. Übersteigt der Patienten-Score diesen Grenzwert, wird eine Empfehlungsmeldung erzeugt, die eine bestimmte Behandlung des Patienten empflehlt. Das Ermitteln des Patienten-Score erfolgt in der Regel durch Aufsummieren einzelner Gewichtungsfaktoren, die beispielsweise vom Geschlecht, Alter oder auch dem Blutdruck abhängen.

Danach werden in Schritt 360 die Daten in einer Datenbank abgespeichert.

Im Nachfolgenden werden die Patientendaten kategorienweise an eine Zielsoftware übergeben. In Schritt 365 werden die Daten einer Kategorie zur Übergabe an eine Patientenverwaltungssoftware vorbereitet. Hierzu werden die Daten in Tastatur-Scan-Codes unter Benutzung einer Scan-Code-Tabelle umgewandelt. Schritt 365 umfasst in manchen Ausführungsformen, obwohl dies in der Figur nicht gezeigt ist, auch das Zwischenpuffem der Tastatur-Scan-Codes in einem Zwischenpufferspeicher. In manchen Ausführungsformen fließt in Schritt 365 des weiteren die ID des Eingabefelds, in das die Eingabe erfolgen soll, mit ein, um sicherzustellen zu können, dass die Eingabe unter Verwendung des richtigen Eingabefelds erfolgt. Diese ID wird hierzu von der Tastatur-Scan-Code-Tabelle abgefragt. Dies kann beispielsweise dadurch erreicht werden, dass die ID in der Tastatur-Scan-Code-Tabelle mit der ID des aktuellen Eingabefeldes verglichen wird.

In Schritt 370 findet die Übergabe der vorbereiteten Daten statt. In Schritt 375 wird überprüft, ob während der Übergabe der Daten ein Fehler aufgetreten ist. Ist dies der Fall, wird überprüft in Schritt 380, ob eine Fokusänderung aufgetreten ist. Wenn dies der Fall war, wird in Schritt 385 der Fokus korrigiert oder zurückgesetzt. Wenn dies nicht der Fall war, werden die Daten erneut übergeben. Für den Fall, dass kein Fehler aufgetreten ist, werden die Schritte 380, 385, 390 nicht ausgeführt. In Schritt 395 wird überprüft, ob die Daten aller Kategorien übergeben wurden. Ist dies nicht der Fall, wiederholen sich die Schritte 365 bis 395 entsprechend. Nachdem alle Daten ausgegeben sind, endet das Verfahren.

Um die Sicherheit der Übergabe der Daten zu steigern, können die Daten in dem Zwischenspeicher mit einer Markierung versehen werden, die ergibt, dass die Daten noch nicht übertragen wurden. Solange diese Markierung vorhanden ist, ist ersichtlich, dass die Übergabe noch nicht erfolgreich war, und diese Markierung kann erst nach erfolgreicher Übergabe der Daten wieder entfernt werden. Anhand einer solchen Markierung ist die Übergabe der Daten sowie die Konsistenz der Daten sowohl in der Schnittstelleneinrichtung als auch in dem PVS-Programm gewährleistet. Dies hat den Vorteil, dass bei einem eventuellen Stromausfall mit einer einhergehenden Unterbrechung der Datenübertragung die Schnittstelleneinrichtung in der Lage ist, nachzuvollziehen, welche Daten vollständig übertragen wurden, und bei welchem Datensatz einer bestimmten Kategorie der Fehler aufgetreten ist. Dieser Vorteil bezieht sich nicht nur auf Stromausfälle, sondern auch auf einfachere Übertragungsfehler, beispielsweise durch eine fehlerhafte Kabelverbindung zwischen der Eingabeschnittstelle und der Programmschnittstelle API des Computergerätes, welches die PVS-Software ausführt.

## Patentansprüche

1. Patientenverwaltungssystem, umfassend:
ein Gerät (230), welches ein Patientenverwaltungssystem-Programm ausführt;
eine Datenquelle (210) zum Bereitstellen medizinischer Daten; und
eine Schnittstelleneinrichtung (100; 220), die zwischen dem Gerät (230) und der Datenquelle (210) eingebunden ist , wobei die Schnittstelleneinrichtung die medizinischen Daten von der Datenquelle (210) empfängt, in Tastatur-Scan-Codes umwandelt, die für das Patientenverwaltungssystem-Programm spezifisch sind, und diese Tastatur-Scan-Codes an das Gerät (230) ausgibt, sowie die vom Gerät (230) empfangenen Daten in einer Datenbank speichert, wobei die Schnittstelleneinrichtung Kategorien der medizinischen Daten anhand Informationen, die zusammen mit den medizinischen Daten übertragen werden, ermittelt, wobei die Kategorien Anamnesen, Befunde, Diagnosen, ICD Codierungen, Therapien, Abrechnungsziffern oder Formulare umfassen, wobei die Schnittstelleneinrichtung mindestens einen Teil der von der Datenquelle (210) empfangenen Daten anhand von Regeln überprüft, wobei die Prüfung des mindestens einen Teils der empfangenen Daten kategorienweise durchgeführt wird und wobei das Ausgeben der Tastatur-Scan-Codes an das Gerät (230) kategorienweise ausgeführt wird .

2. Patientenverwaltungssystem gemäß Anspruch 1, wobei die Datenquelle (210) ein medizinisches Gerät ist, und wobei das medizinische Gerät ein Röntgen-Gerät oder ein Ultraschall-Untersuchungsgerät ist, oder wobei die Datenquelle (210) eine grafische Eingabeschnittstelle ist.

3. Patientenverwaltungssystem gemäß einem der Ansprüche 1 oder 2, wobei die Schnittstelleneinrichtung (100) über eine Schnittstelle für den Transfer von medizinischen Daten mit der Datenquelle (210) verbunden ist, und wobei die Schnittstelle für den Transfer von medizinischen Daten eine GDT-Schnittstelle (105), eine BDT-Schnittstelle oder eine HL7-Schnittstelle ist.

4. Patientenverwaltungssystem gemäß einem der Ansprüche 1 bis 3, wobei die Umwandlung in Tastatur-Scan-Codes anhand einer Zuordnungstabelle geschieht, wobei die Zuordnungstabelle angibt, in welche Tastatur-Scan-Codes die von der Datenquelle (210) empfangenen Daten in Abhängigkeit von dem verwendeten Patientenverwaltungssystem-Programm umgewandelt werden.

5. Patientenverwaltungssystem gemäß einem der Ansprüche 1 bis 4 wobei das Ausgeben der Tastatur-Scan-Codes an das Gerät (230) ein Injizieren der Tastatur-Scan-Codes in eine API des Geräts (230) umfasst, wobei die Tastatur-Scan-Codes in einem Pufferspeicher zwischengespeichert werden, und wobei bei dem Kategorienweisen Ausgeben der Tastatur-Scan-Codes die aktuell auszugebenden Tastatur-Scan-Codes in dem Pufferspeicher vor der Ausgabe mit einem Marker versehen werden, der anzeigt, dass die Ausgabe noch nicht abgeschlossen ist.

6. Patientenverwaltungssystem gemäß Anspruch 5, wobei die Ausgabe der Tastatur-Scan-Codes an das Gerät (230) wiederholt wird, wenn die Schnittstelleneinrichtung im Zusammenhang mit der Ausgabe der Tastatur-Scan-Codes and das Gerät (230) eine Fehlermeldung von dem Gerät (230) empfängt, wobei die Tastatur-Scan-Codes in einem Pufferspeicher zwischengespeichert werden,
wobei die Wiederholung der Ausgabe der Tastatur-Scan-Codes an das Gerät (230) unter Verwendung der in dem Pufferspeicher zwischengespeicherten Tastatur-Scan-Codes geschieht, und wobei nach der Ausgabe der Tastatur-Scan-Codes an das Gerät (230), wenn die Schnittstelleneinrichtung im Zusammenhang mit der Ausgabe der Tastatur-Scan-Codes and das Gerät (230) keine Fehlermeldung vom Gerät (230) empfängt, der Marker, der anzeigt, dass die Ausgabe noch nicht abgeschlossen ist, wieder gelöscht wird.

7. Patientenverwaltungssystem gemäß einem der Ansprüche 1 bis 6, wobei die Schnittstelleneinrichtung einen Patienten-Score unter Berücksichtigung medizinischer Daten bildet und den gebildeten Patienten-Score mit einem oder mehreren vorgegebenen Score-Werten vergleicht, wobei die Schnittstelleneinrichtung eine Benachrichtigung auslöst, wenn der Patienten-Score einen der vorgegebenen Score-Werte übersteigt, und wobei die Benachrichtigung ein Erzeugen einer Behandlungsempfehlung oder eine Empfehlung zur Überweisung zum Facharzt oder Krankenhaus umfasst.

8. Patientenverwaltungssystem gemäß einem der Anspruch 1 bis 7, wobei die Datenbank zur Speicherung der vom Gerät (230) empfangenen Daten eine externe Datenbank ist, wobei die externe Datenbank auf einem Datenbankserver betrieben wird, und wobei die Datenbank eine SQL-Datenbank ist.

9. Patientenverwaltungssystem gemäß einem der Ansprüche 1 bis 8, wobei die Schnittstelleneinrichtung eingerichtet ist, die vom Gerät (230) empfangenen Daten an ein weiteres Programm auszugeben, wobei die Schnittstelleneinrichtung des Weiteren eingerichtet ist, die in der Datenbank gespeicherten Daten an das weitere Programm auszugeben, wobei die Ausgabe der Daten anonymisiert erfolgt, wobei das weitere Programm eingerichtet ist, um in Erwiderung auf die von der Schnittstelleneinrichtung empfangenen Daten eine Behandlungsempfehlung für den/die Patienten auszugeben, und
wobei das weitere Programm eine Care-Engine ist.

10. Patientenverwaltungssystem gemäß einem der Ansprüche 1 bis 9, wobei die Schnittstelleneinrichtung eingerichtet ist, unter Verwendung einer Datenbank, die Wechselwirkungen zwischen Medikamenten beschreibt, die Daten in einer Kategorie auf mögliche Wechselwirkungen zu prüfen und eine Warnung auszugeben, wenn eine mögliche Wechselwirkung festgestellt wird.

11. Schnittstelleneinrichtung, die eingerichtet ist, um zwischen einem Gerät, welches ein Patientenverwaltungssystem-Programm ausführt, und einer Datenquelle zum Bereitstellen medizinischer Daten eingebunden zu werden, wobei die Schnittstelleneinrichtung die medizinischen Daten von der Datenquelle empfängt, in Tastatur-Scan-Codes umwandelt, die für das Patientenverwaltungssystem-Programm spezifisch sind, und diese Tastatur-Scan-Codes an das Gerät ausgibt, sowie die von dem Gerät empfangenen Daten in einer Datenbank speichert, wobei die Schnittstelleneinrichtung Kategorien der medizinischen Daten anhand Informationen, die zusammen mit den medizinischen Daten übertragen werden, ermittelt, wobei das Ausgeben der Tastatur-Scan-Codes an das Gerät (230) kategorienweise ausgeführt wird, wobei die Schnittstelleneinrichtung mindestens einen Teil der von der Datenquelle (210) empfangenen Daten anhand von Regeln überprüft, wobei die Prüfung des mindestens einen Teils der empfangenen Daten kategorienweise durchgeführt wird und wobei die Kategorien Anamnesen, Befunde, Diagnosen, ICD Codierungen, Therapien, Abrechnungsziffern oder Formulare umfassen.

12. Schnittstelleneinrichtung gemäß Anspruch 11, wobei die Schnittstelleneinrichtung angepasst ist, um gemäß einem der Ansprüche 2 bis 10 betrieben zu werden.

13. Patientenverwaltungsverfahren zum Betreiben einer Schnittstelleneinrichtung, welche mit einem Gerät, welches ein Patientenverwaltungssystem-Programm ausführt, verbindbar ist, wobei das Verfahren die folgenden Schritte umfasst:
Empfangen (320) von medizinischen Daten von einer Datenquelle zum Bereitstellen medizinischer Daten;
Umwandeln (365) der medizinischen Daten in Tastatur-Scan-Codes, die für das Patientenverwaltungssystem-Programm spezifisch sind;
Speichern (360) der von der Datenquelle empfangenen Daten in einer Datenbank;
Ermitteln von Kategorien der medizinischen Daten anhand Informationen, die zusammen mit den medizinischen Daten übertragen werden, wobei die Kategorien Anamnesen, Befunde, Diagnosen, ICD Codierungen, Therapien, Abrechnungsziffern oder Formulare umfassen;
Überprüfen von mindestens einem Teil der von der Datenquelle empfangenen medizinischen Daten anhand von Regeln, wobei die Prüfung des mindestens einen Teils der medizinischen Daten kategorienweise ausgeführt wird; und
Ausgeben (370) der Tastatur-Scan-Codes an das Gerät, wobei das Ausgeben kategorienweise erfolgt.

## Claims

1. A patient administration system, comprising:
a device (230) which executes a patient administration system program;
a data source (210) for making medical data available; and
an interface device (100; 220) which is coupled between the device (230) and the data source (210), wherein the interface device receives the medical data from the data source (210), converts them into keyboard scan codes which are specific to the patient administration system program and outputs said keyboard scan codes to the device (230) and stores the data received from the device (230) in a database, wherein the interface device ascertains categories of the medical data on the basis of information transmitted together with the medical data, wherein the categories include medical histories, results, diagnoses, ICD codes, therapies, accounting items or forms, wherein the interface device checks at least part of the data received from the data source (210) by means of rules, wherein said at least part of the received data is checked categorywise and wherein the keyboard scan codes are outputted to the device (230) categorywise.

2. The patient administration system according to claim 1, wherein the data source (210) is a medical device and wherein the medical device is an X-ray device or an ultrasonic examination device or wherein the data source (210) is a graphic input interface.

3. The patient administration system according to claim 1 or 2, wherein the interface device (100) is connected to the data source (210) via an interface for the transfer of medical data and wherein the interface for the transfer of medical data is a GDT interface (105), a BDT interface or an HL7 interface.

4. The patient administration system according to any of claims 1 to 3, wherein the conversion into keyboard scan codes is done on the basis of a cross reference list, wherein the cross reference list indicates the keyboard scan codes into which the data received from the data source (210) are converted depending on the patient administration system program used.

5. The patient administration system according to any of claims 1 to 4, wherein outputting of the keyboard scan code to the device (230) includes injecting the keyboard scan codes into an API of the device (230), wherein the keyboard scan codes are buffered in a buffer storage, and wherein during the categorywise output of the keyboard scan codes the keyboard scan codes currently to be outputted are marked by a marker in the buffer storage before they are outputted, said marker indicating that the output has not yet been terminated.

6. The patient administration system according to claim 5, wherein outputting of the keyboard scan codes to the device (230) is repeated, if the interface device receives an error message from the device (230) with regard to the output of the keyboard scan codes to said device (230), wherein the keyboard scan codes are buffered in a buffer storage, wherein outputting of the keyboard scan codes to the device (230) is repeated by making use of the keyboard scan codes buffered in said buffer storage, and wherein after outputting the keyboard scan codes to the device (230), in case the interface device does not receive an error message from the device (230) with regard to the output of the keyboard scan codes to the device (230), the marker indicating that the output has not yet been terminated is canceled.

7. The patient administration system according to any of claims 1 to 6, wherein the interface device creates a patient score with due regard to medical data and compares said created patient score to one or several predefined score values, wherein the interface device releases a message if the patient score exceeds one of said predefined score values, and wherein said message includes a creation of a recommendation of treatment or a recommendation for referral to a specialist or a hospital.

8. The patient administration system according to any of claims 1 to 7, wherein the database for storing the data received from said device (230) is an external database, wherein the external database is run on a database server, and wherein said database is an SQL database.

9. The patient administration system according to any of claims 1 to 8, wherein the interface device is adapted to output the data received from the device (230) to another program, wherein the interface device is further adapted to output the data stored in the database to the additional program, wherein said data are output in anonymous form, wherein the additional program is adapted to output a recommendation of treatment for the patient(s) in response to the data received from the interface device, and wherein said other program is a Care engine.

10. The patient administration system according to any of claims 1 to 9, wherein the interface device is adapted to check, by making use of a database that describes interactions between medications, the data in one category with regard to possible interactions and to output a warning if any possible interactions should be detected.

11. An interface device adapted to be integrated between a device which executes a patient administration system program and a data source for making medical data available, wherein the interface device receives the medical data from the data source, converts them into keyboard scan codes which are specific to the patient administration system program and outputs said keyboard scan codes to the device and stores the data received from the device in a database, wherein the interface device ascertains categories of the medical data on the basis of information transmitted together with the medical data, wherein the keyboard scan codes are outputted to the device (230) categorywise, wherein the interface device checks at least part of the data received from the data source (210) by means of rules, wherein said at least part of the received data is checked categorywise and wherein the categories include medical histories, results, diagnoses, ICD codes, therapies, accounting items or forms.

12. The interface device according to claim 11, wherein the interface device is adapted to be operated in accordance with any of claims 2 to 10.

13. A patient administration method for operating an interface device that is connectable to a device which executes a patient administration system program, the method comprising the following steps:
receiving (320) medical data from a data source for making medical data available;
converting (365) the medical data into keyboard scan codes which are specific to the patient administration system program;
storing (360) the data received from the data source in a database;
ascertaining categories of the medical data on the basis of information transmitted together with the medical data, wherein the categories include medical histories, results, diagnoses, ICD codes, therapies, accounting items or forms;
checking at least part of the medical data received from the data source by means of rules, wherein said at least part of the medical data is checked categorywise; and outputting (370) the keyboard scan codes to the device, said outputting being executed categorywise.

## Revendications

1. Système de gestion de patients, comprenant :
un appareil (230), qui exécute un programme de système de gestion de patients;
une source de données (210) destinée à fournir des données médicales; et
un dispositif d'interface (100; 220), qui est intégré entre l'appareil (230) et la source de données (210), système
dans lequel le dispositif d'interface reçoit les données médicales de la source de données (210), les convertit en scan-codes de clavier, qui sont spécifiques au programme de système de gestion de patients, et délivre ces scan-codes de clavier à l'appareil (230), et stocke également les données reçues par l'appareil (230) dans une banque de données,
dans lequel le dispositif d'interface détermine des catégories des données médicales au regard d'informations, qui sont transmises en commun avec les données médicales,
dans lequel les catégories englobent des anamnèses, des résultats ou rapports médicaux, des diagnostics, des codages ICD, des thérapies, des données comptables chiffrées ou des formulaires,
dans lequel le dispositif d'interface vérifie au moins une partie des données reçues de la source de données (210), au regard de règles,
dans lequel la vérification de ladite au moins une partie des données reçues s'effectue par catégories, et dans lequel la délivrance des scan-codes de clavier à l'appareil (230) s'effectue par catégories.

2. Système de gestion de patients selon la revendication 1,
dans lequel la source de données (210) est un appareil médical, et dans lequel l'appareil médical est un appareil de radiographie ou un appareil d'examen par ultrasons, ou bien
dans lequel la source de données (210) est une interface de saisie graphique.

3. Système de gestion de patients selon la revendication 1 ou la revendication 2,
dans lequel le dispositif d'interface (100) est relié à la source de données (210) par une interface pour le transfert de données médicales, et
dans lequel l'interface pour le transfert de données médicales est une interface GDT (105), une interface BDT ou une interface HL7.

4. Système de gestion de patients selon l'une des revendications 1 à 3,
dans lequel la conversion en scan-codes de clavier se fait au regard d'un tableau d'affectation, et
dans lequel le tableau d'affectation indique en quels scan-codes de clavier vont être converties les données reçues à partir de la source de données (210), en fonction du programme de système de gestion de patients utilisé.

5. Système de gestion de patients selon l'une des revendications 1 à 4,
dans lequel la délivrance des scan-codes de clavier à l'appareil (230) comprend une injection des scan-codes de clavier dans une API de l'appareil (230),
dans lequel les scan-codes de clavier sont stockés temporairement dans une mémoire tampon, et
dans lequel lors de la délivrance par catégories des scan-codes de clavier, les scan-codes de clavier dans la mémoire tampon, à délivrer actuellement, sont munis d'un marqueur qui indique que la délivrance n'est pas encore achevée.

6. Système de gestion de patients selon la revendication 5,
dans lequel la délivrance des scan-codes de clavier à l'appareil (230) est répétée lorsque le dispositif d'interface, en relation avec la délivrance des scan-codes de clavier à l'appareil (230), reçoit un message d'erreur de l'appareil (230),
dans lequel les scan-codes de clavier sont stockés temporairement dans une mémoire tampon, dans lequel la répétition de la délivrance des scan-codes de clavier à l'appareil (230) se fait en utilisant les scan-codes de clavier stockés temporairement dans la mémoire tampon,
et
dans lequel après délivrance des scan-codes de clavier à l'appareil (230), si le dispositif d'interface, en relation avec la délivrance des scan-codes de clavier à l'appareil (230), ne reçoit pas de message d'erreur de l'appareil (230), le marqueur, qui indique que la délivrance n'est pas encore achevée, est à nouveau effacé.

7. Système de gestion de patients selon l'une des revendications 1 à 6,
dans lequel le dispositif d'interface forme un score-patient en tenant compte de données médicales, et compare le score-patient formé avec une ou plusieurs valeurs de score prescrites,
dans lequel le dispositif d'interface produit une notification d'information lorsque le score-patient dépasse l'une des valeurs de score prescrites, et
dans lequel la notification d'information comprend l'élaboration d'une recommandation de traitement ou d'une recommandation pour l'envoi chez un médecin spécialiste ou à l'hôpital.

8. Système de gestion de patients selon l'une des revendications 1 à 7,
dans lequel la banque de données pour le stockage des données reçues par l'appareil (230) est une banque de données extérieure,
dans lequel la banque de données extérieure fonctionne sur un serveur de banque de données, et
dans lequel la banque de données est une banque de données SQL.

9. Système de gestion de patients selon l'une des revendications 1 à 8,
dans lequel le dispositif d'interface est configuré pour délivrer les données reçues par l'appareil (230) à un autre programme,
dans lequel le dispositif d'interface est par ailleurs configuré pour délivrer les données stockées dans la banque de données, au dit autre programme,
dans lequel la délivrance des données s'effectue de manière anonymisée,
dans lequel ledit autre programme est configuré pour délivrer, en réponse aux données reçues par le dispositif d'interface, une recommandation de traitement pour le/les patients, et
dans lequel ledit autre programme est un programme dit care-engine.

10. Système de gestion de patients selon l'une des revendications 1 à 9,
dans lequel le dispositif d'interface est configuré pour, par l'utilisation d'une banque de données, qui décrit des interactions entre médicaments, examiner les données dans une catégorie, quant à de possibles interactions, et émettre une alerte lorsqu'une interaction possible a été constatée.

11. Dispositif d'interface, qui est conçu pour être intégré entre un appareil, qui exécute un programme de système de gestion de patients, et une source de données fournissant des données médicales, ensemble dans lequel le dispositif d'interface reçoit les données médicales de la source de données, les convertit en scan-codes de clavier, qui sont spécifiques au programme de système de gestion de patients, et délivre ces scan-codes de clavier à l'appareil, et stocke également les données reçues par l'appareil dans une banque de données,
dans lequel le dispositif d'interface détermine des catégories des données médicales au regard d'informations, qui sont transmises en commun avec les données médicales,
dans lequel la délivrance des scan-codes de clavier à l'appareil (230) s'effectue par catégories,
dans lequel le dispositif d'interface vérifie au moins une partie des données reçues de la source de données (210), au regard de règles,
dans lequel la vérification de ladite au moins une partie des données reçues s'effectue par catégories, et dans lequel les catégories englobent des anamnèses, des résultats ou rapports médicaux, des diagnostics, des codages ICD, des thérapies, des données comptables chiffrées ou des formulaires.

12. Dispositif d'interface selon la revendication 11,
le dispositif d'interface étant adapté à pouvoir fonctionner conformément à l'une des revendications 2 à 10.

13. Procédé de gestion de patients pour faire fonctionner un dispositif d'interface, qui peut être relié à un appareil exécutant un programme de système de gestion de patients, le procédé comprenant les étapes suivantes :
réception (320) de données médicales à partir d'une source de données destinée à fournir des données médicales ;
conversion (365) des données médicales en scan-codes de clavier, qui sont spécifiques au programme de système de gestion de patients,
stockage (360) des données reçues à partir de la source de données, dans une banque de données ;
détermination de catégories des données médicales au regard d'informations, qui sont transmises en commun avec les données médicales, les catégories englobant des anamnèses, des résultats ou rapports médicaux, des diagnostics, des codages ICD, des thérapies, des données comptables chiffrées ou des formulaires ;
vérification d'au moins une partie des données médicales reçues de la source de données, au regard de règles, la vérification de ladite au moins une partie des données médicales étant effectuée par catégories ; et
délivrance (370) des scan-codes de clavier à l'appareil, la délivrance étant effectuée par catégories.
